# EUROPÄISCHE PATENTSCHRIFT

(11) **EP 3 237 076 B1**
(45) Veröffentlichungstag und Bekanntmachung des Hinweises auf die Patenterteilung: **13.02.2019**
(21) Anmeldenummer: 15817829.3
(22) Anmeldetag: 21.12.2015
(51) Int. Cl.: A62B 7/14, A61M 16/10, A61K 33/00, B64D 25/00

(54) **GASGEMISCH SOWIE SEINE VERWENDUNG ZUR BEDARFSWEISEN BEATMUNG VON MENSCHEN BEI DRUCKABFÄLLEN IN FLUGZEUGEN UND VERFAHREN DAZU**
GAS MIXTURE AND ITS USE FOR BREATHING OF HUMANS DURING PRESSURE DROPS IN AIRPLANES, AND CORRESPONDING METHODS
MÉLANGE DE GAZ ET SON UTILISATION POUR LA RESPIRATION DE PERSONNES SI BESOIN EST EN CAS DE CHUTES DE PRESSION DANS DES AVIONS ET PROCÉDÉ ASSOCIÉ

(30) Priorität: 24.12.2014 CH 20282014
(43) Veröffentlichungstag der Anmeldung: 01.11.2017
(73) Patentinhaber: Caeli Nova AG, 8808 Pfäffikon SZ (CH)
(72) Erfinder: STUDER, Marc, 8164 Bachs (CH)
(74) Vertreter: EGLI-EUROPEAN PATENT ATTORNEYS
(86) Internationale Anmeldenummer: PCT/EP2015/080770
(87) Internationale Veröffentlichungsnummer: WO 2016/102450

(56) Entgegenhaltungen:
- WO-A1-2007/121773
- CH-A1- 702 633
- US-A- 4 282 870
- US-A- 5 460 175
- US-A- 5 647 345

## Beschreibung

Die Erfindung betrifft ein Gasgemisch als Erzeugnis, sowie die Verwendung dieses Gasgemisches zur bedarfsweisen Beatmung von Menschen in grossen Dichtehöhen. Ausserdem betrifft die Erfindung ein Verfahren, um das Gasgemisch für die Beatmung verfügbar zu machen. Im Besonderen geht es um die Verwendung eines bestimmten Gasgemisches als Erzeugnis für die Beatmung bei Druckabfällen in Flugzeugen zur Sicherstellung einer ausreichenden Sauerstoffsättigung der Insassen sowie zur Unterstützung bei unzureichender oder fehlender Spontanatmung bzw. Hyperventilation.

Dem menschlichen Körper ist je nach Alter, Situation und Krankheitsbild ein typischer Sauerstoffsättigungswert eigen. Dieser gibt den Anteil O₂-beladenen Hämoglobins im Blut an, was Aufschluss über die Effizienz der Atmung und des O₂-Transports im Körper gibt. Sauerstoffuntersättigung ergibt sich entweder aufgrund eines für den Menschen zu geringen Sauerstoffpartialdruckes in der Umgebung (etwa in einer Höhe über 10'000ft bzw. 3048m) und/oder als Folge gesundheitlicher Störungen. Somit variieren auch die Behandlungsmassnahmen. Grundsätzlich unterscheidet man bei der Beatmung zwischen einer assistierten Beatmung und einer kontrollierten (mandatorischen) Beatmung. Dabei wirkt ein Beatmungsgerät im Fall der assistierten Beatmung rein unterstützend aufgrund ungenügender Spontanatmung. Der Patient atmet selbst und steuert die Atemfrequenz. Im Gegensatz dazu ersetzt das Beatmungsgerät bei der kontrollierten Beatmung die körpereigene Atemfunktion vollständig. Die O₂-Konzentration der künstlich zugeführten Luft ist je nach Bedarf zwischen einer Normalkonzentration von 21% bis zu 100% am Gasgemisch einstellbar. Mit FiO₂ (*fraction of inspired oxygen*) wird der inspiratorische Sauerstoffanteil bezeichnet. Bekannt ist, dass ein FiO₂ von über 0.5 (entspricht einem 50%igen Sauerstoffanteil in der Atemluft) über längere Zeit verabreicht, schädigend wirkt. Sauerstoff ist nämlich ein starkes Oxidationsmittel, das neben Hämoglobin auch andere im Blut vorkommende Stoffe oxidiert. Enzyme im Körper machen diesen Oxidationsprozess jedoch wieder rückgängig. Wird einem Körper hingegen über eine bestimmte Dauer hinaus reiner Sauerstoff zugeführt, kann die sogenannte Methämoglobinreduktase angesichts der geförderten Oxidation von Hämoglobin und anderen Proteinen diesen Schaden nicht mehr beheben. Die freiwerdenden Sauerstoffradikale führen nach Erschöpfung des körpereigenen Antioxidationssystems zu einer Sauerstoffvergiftung mit Symptomatik am zentralen Nervensystem, an der Lunge und an den Augen. Dennoch werden Patienten in lebensbedrohlichem Zustand - wenn auch nur kurzzeitig - mit reinem Sauerstoff, das heisst bei FiO₂=1, beatmet. Auch bei der Präoxygenierung, der vorbeugenden Anreicherung des Sauerstoffspeichers der Lunge, beispielsweise vor einer Narkoseeinleitung, wird dem Patienten 100%iger Sauerstoff zugeführt, damit der sich in der Atemluft befindliche Stickstoff aus den Atemwegen ausgewaschen wird. Ebenfalls mit reinem Sauerstoff beatmet werden Crew und Passagiere im Fall eines Kabinendruckabfalls. Hier überwiegt das Argument, dass eine grosse Menge Sauerstoff schnellstmöglich ins evtl. bereits unterversorgte Körpergewebe geführt werden soll. Allfällige Nebenwirkungen werden daher ausgeblendet bzw. in Kauf genommen.

Allerdings sind die Gefahren bei der Beatmung nicht allein den Eigenschaften des Sauerstoffs zuzuschreiben. Eine wesentliche Rolle spielt auch die CO₂-Konzentration im (arteriellen) Blut. Die Atemsteuerung und -regulierung erfolgt vorwiegend über Chemorezeptoren bzw. Chemosensoren, welche für den CO₂-Partialdruck sensibel sind (sauerstoff-sensible oder andere Rezeptoren spielen hierfür eine untergeordnete Rolle). Der CO₂-Pegel im Blut ist daher ein vegetativer Stimulus zur Regulierung der Atmung. Übersteigt der CO₂-Gehalt im Blut einen charakteristischen Schwellenwert, setzt der Atemreiz ein. Umgekehrt wird bei der Hyperventilation und der damit einhergehenden Abnahme des CO₂-Partialdrucks im Blut (Hypokapnie) die Atmung reflektorisch begrenzt. Um den Atemantrieb zu unterdrücken, hyperventilieren ungeübte Taucher daher oftmals unbesehen, um CO₂ auszuatmen und sodann länger unter Wasser zu verbleiben. Dies ist jedoch mit erheblichen Risiken behaftet, welche bis zu Bewusstlosigkeit und -diesfalls- Ertrinken führen können (sogenanntes Schwimmbad-Blackout). Mit sinkendem körpereigenem Kohlenstoffdioxidgehalt können Mangelerscheinungen als unangenehme bis lebensbedrohliche Symptome auftreten, wie in Abschnitt **[0025]** im Einzelnen beschrieben. Der CO₂-pegelsenkende Effekt einer Hyperventilation wird bei mangelnder Bewegungsfreiheit noch verstärkt, weil die Muskeln in dieser Situation weniger körpereigenes Kohlendioxid erzeugen. Die Symptome eines Kohlendioxidmangels treten so schneller und verstärkt ein. Diese Problematik tritt insbesondere bei Druckabfällen in einer Flugzeugkabine auf, weil die Passagiere dann zwangsläufig bewegungseingeschränkt sind. Der CO₂-Partialdruck bedarf während des Beatmungsvorgangs in einer solchen Situation notwendigerweise einer Überprüfung, etwa durch Messung des endexspiratorischen CO₂-Partialdrucks.

Fällt der Druck in einer Flugzeugkabine unter einen kritischen Druckwert ab, öffnen sich die Fächer über den Sitzplätzen und die in der Kabinendecke befindlichen Sauerstoffmasken fallen aus ihren Halterungen und vor das Gesichtsfeld der Flugzeuginsassen. Durch den zuführenden Schlauch strömt reiner Sauerstoff in die Maske und gelangt durch Einatmen schliesslich in Nase und Mund des Atmenden. Für die Versorgung der Passagiere wird Sauerstoff entweder in chemischen Sauerstoffgeneratoren erzeugt oder in Druckflaschen mitgeführt, während die Piloten im Cockpit über ein separates System mit Druckgas-Sauerstoff versorgt werden. Wie gross der Sauerstoffvorrat in einem Flugzeug ist, hängt von dessen Zertifizierung und dessen Einsatzzweck sowie der zu fliegenden Flugstrecken ab. Dabei wird berücksichtigt, ob das Flugzeug mehrheitlich Land oder Meer überfliegt, oder ob es gar lange Distanzen über sehr hochgelegene Gebiete und Hochgebirgszüge fliegt.

Die Zeitspanne, die bei einem Druckabfall in der Flugzeugkabine zum sinnvollen Handeln verbleibt, bezeichnet man als *time of useful consciousness* (TUC) oder *Effective Performance Time* (EPT). Danach tritt eine deutliche Sauerstoffunterversorgung des Körpergewebes und der Organe ein und der Körper wird hypoxisch. Unter einer bestimmten Sauerstoffsättigung des Gehirns verliert man die Handlungsfähigkeit und später das Bewusstsein. Die TUC wird für eine Flugfläche

(Fläche gleichen Luftdrucks in der Atmosphäre, entsprechend einer bestimmten Flughöhe in Hektofuss) angegeben und verringert sich mit zunehmender Flughöhe. Untenstehende Tabelle gibt TUC-Angaben für verschiedene Flugflächen an.

| **Flugfläche** | **TUC** | **Höhe in Metern** | **Höhe in Fuss** |
|---|---|---|---|
| FL 150 | ≥ 30 min | 4,572 | 15,000 |
| FL 180 | 20 - 30 min | 5,486 | 18,000 |
| FL 220 | 5 - 10 min | 6,705 | 22,000 |
| FL 250 | 3 - 6 min | 7,620 | 25,000 |
| FL 280 | 2.5 - 3 min | 8,534 | 28,000 |
| FL 300 | 1 - 3 min | 9,144 | 30,000 |
| FL 350 | 30 - 60 sec | 10,668 | 35,000 |
| FL 400 | 15 - 20 sec | 12,192 | 40,000 |
| FL 430 | 9 - 15 sec | 13,106 | 43,000 |
| ≥ FL 500 | 6 - 9 sec | 15,240 | 50,000 |

Die Geschwindigkeit des Dekompressionseintritts hat ebenfalls einen Einfluss auf die TUC. Je schneller der Druckabfall von statten geht, desto kleiner ist die TUC. Deshalb ist angesichts der rasch eintretenden Sauerstoffunterversorgung des Körpers eine schnellstmögliche Versorgung mit Sauerstoff überlebenswichtig, weshalb Militärpiloten, welche auch oberhalb des Passagierflugverkehrs fliegen, ihre Sauerstoffmasken während des gesamten Fluges einsatzbereit tragen. Für die Zivilluftfahrt besteht keine solche Notwendigkeit, da die Reiseflughöhe von Verkehrsflugzeugen tiefer liegt, nämlich zwischen FL 250 und FL 450, was einer Flughöhe von rund 25'000ft bis 40'000ft entspricht.

Aus kommerziellen Gründen sollten Flugstrecken idealerweise auf kürzestem Weg zwischen zwei Flughäfen geflogen werden. Heutzutage erlauben die Reichweiten moderner Verkehrsflugzeuge (inter-)kontinentale Direktflüge zu Destinationen, die bis vor wenigen Jahren nur via Zwischenstopps erreichbar waren. Aus sicherheitstechnischen Gründen aber sind nicht alle direkten Routen für den Flugverkehr freigegeben. Das Befliegen von hochgelegenen Gebirgszügen wie dem Himalaya zwischen Indien und Tibet, dem zentralasiatischen Hindukusch-Gebirge und den südamerikanischen Anden ist nur beschränkt und unter gewissen Voraussetzungen möglich. Ausschlaggebend dafür sind zwei zu unterscheidende Notfallszenarien: Erstens Triebwerkausfall und zweitens Druckabfall in der Kabine. Im ersten Fall besteht die Gefahr im Schubverlust durch den Ausfall eines oder mehrerer Triebwerke, was eine Reduktion der Flughöhe erzwingt, weil das Flugzeug mit verringertem Schub die Reisehöhe nicht halten kann. Abhilfe schaffen Fluchtrouten, über welche ein *drift-down* zur nächstmöglichen Landepiste eingeleitet werden kann. Die ICAO (International Civil Aviation Organization), die EASA (European Aviation Safety Agency), die JAA (Joint Aviation Authorities) sowie die FAA (U.S. Federal Aviation Administration) schreiben für solche Fluchtwege als Standard vor, dass innerhalb einer spezifischen horizontalen Bandbreite zur Flugstrasse ein Vertikalabstand von wenigstens 2'000ft zum Boden während eines *engine-out drift-down* Manövers bis zur *OEI (one-engine inoperable) service ceiling* und bei sich einpendelnder Flughöhe ein Vertikalabstand von 1'000ft zum Boden bzw. 2000ft über Gebirgsterrain gegeben sein muss. Was die Breite der besagten Hindernisfreigrenzen betrifft, unterscheiden sich die Vorschriften der Regulierungsstellen.

Durch das ausgeklügelte System der Fluchtrouten wären allein aufgrund von triebwerkausfallbedingten Notlandungen beliebige Direktrouten mit evtl. geringfügigen Abweichungen realisierbar. Doch der zweite Fall eines möglichen Notfallszenarios, nämlich eines Dekompressionsvorfalls in der Kabine, stellt ein wesentlich restriktiveres Problem dar. Zusätzlich zu den oben genannten Bedingungen ist der Faktor Zeit hierbei viel limitierender als bei einem Triebwerkausfall. Die Anzahl prinzipiell möglicher Fluchtwege reduziert sich, weil auf vielen solchen die erforderliche Höhendifferenz nicht innerhalb einer genügend kleinen Zeitspanne erreicht werden kann. Die Standard-Prozedur bei Druckverlust in der Flugzeugkabine ist durch die ICAO reglementiert. Sie sieht vor, dass die Piloten - nachdem sie durch ihre eigene Sauerstoffmaske atmen - schnellstmöglich einen Sinkflug einleiten und das Flugzeug auf eine sichere Höhe bringen, also auf eine Höhe, in der ein Mensch ohne zusätzliche Sauerstoffzufuhr atmen kann. Dies muss innerhalb jener Zeitspanne erfolgen, die der Sauerstoffvorrat als Überbrückungszeit vorgibt. Aufgrund der beschränkten Raum- und Gewichtskapazität der Flugzeuge lässt sich der Sauerstoffvorrat nur auf Kosten der mitgeführten Fracht oder der maximalen Anzahl Passagiere erhöhen. Eine verbesserte Beatmung der Flugzeuginsassen bei Dekompressionsvorfällen könnte auch dazu führen, dass weniger Sauerstoff mitgeführt werden muss und sich dadurch das Flugzeuggewicht verringern würde.

Wäre das Beatmungsproblem inexistent, so könnten Verkehrsflugzeuge in vielen Fällen auf einer direkteren Route zu ihren Destinationen fliegen. Wäre nur der Triebwerkausfall massgebend, könnten mit entsprechenden mehrstrahligen Flugzeugen beliebige Gebiete mit Passagieren überflogen werden, da das Fluchtwegsystem auf jedem Streckenabschnitt einen *drift-down* bis zur *one-engine inoperable service ceiling* zulässt. Das System der Fluchtrouten ist in der Praxis jedoch so ausgelegt, dass die verschiedenen Fluchtwege dem anforderungsreicheren der beiden oben genannten Notfallszenarien - demjenigen einer spontanen Dekompression - genügen. Eine verbesserte Beatmung der Flugzeuginsassen bei Dekompressionsvorfällen in der Kabine, die mit einem geringeren Sauerstoffbedarf auskäme, würde das durch den üblichen Sauerstoffvorrat vorgegebene Zeitintervall verlängern. Somit müssten unter Umständen auch keine über einen Triebwerkausfall hinausgehenden routentechnischen Sicherheitsvorkehrungen getroffen werden. Ohne diese zusätzlichen Einschränkungen könnten Reiserouten über Hochterrain ohne Umwege geflogen und in der Folge Tausende von Tonnen Kerosin eingespart werden. Durch das eingesparte Gewicht an Treibstoff könnte entweder die Ladekapazität erhöht oder aber der Treibstoffverbrauch insgesamt reduziert werden, da das Flugzeug um das Eigengewicht des überflüssigen Treibstoffs leichter wird. Letzteres würde auch zur Schonung der Umwelt beitragen. Daneben könnte erhebliche Flugzeit eingespart werden, was nicht zuletzt operationell viele Vorteile bietet und überdies den Anflug weiter entfernter Destinationen ermöglicht.

Die heutzutage aktiv beflogenen Flugrouten über ausgedehntes Hochgebirgsterrain müssen mit erheblichem Aufwand geplant und durchgeführt werden. Bei grösseren technischen Problemen und Störungen liegt die effektive Abwicklung der Notmassnahmen vor allem in den Händen der Piloten, welche sofortige Entscheidungen treffen und ihre unmittelbare Umsetzung veranlassen müssen. Weil Entscheidungen in solchen Situationen meistens irreversibel sind, ist der Lauf der Dinge zum grossen Teil von Menschen bestimmt, welche in diesen Stresssituationen reagieren müssen. Damit aber ist ein wesentliches Risiko für Fehler mit evtl. schwerwiegenden Folgen a priori gegeben. Ein weniger dominanter Zeitfaktor bzw. eine längere Entscheidungsfrist in Notsituationen würde die Qualität der Entscheidungen um einiges erhöhen und damit wesentlich zur Sicherheit beitragen.

Die WO 2007/121 773 A1 beschreibt ein Sauerstoffversorgungssystem für Flugzeuge, bei dem die Sauerstoffkonzentration in einer im Notfall bereitgestellten Gasmischung zum Erzielen einer Sauerstoffvorsättigung mit zunehmender Flughöhe erhöht wird.

Aus der US 5 647 345 A ist ein Atemstimulationsgerät bekannt, bei welchem zur Vermeidung von Akapnie ausgeatmete Luft mit neu zugeführter Frischluft gemischt wird, um so eine hinreichende CO₂-Konzentration im Blut und damit den Atemreflex aufrechtzuerhalten.

Die Aufgabe der vorliegenden Erfindung besteht folglich darin, ein Erzeugnis und generell ein Verfahren sowie eine Verwendung dieses Erzeugnisses zur bedarfsweisen Beatmung in grossen Dichtehöhen oder bei Hyperventilation anzugeben, zur Sicherstellung der Beatmung und zur Verbesserung von Körperfunktions- und Leistungsfähigkeit mittels einer effizienteren Beatmung, wenn eine solche nötig wird. Weiter besteht die Aufgabe der Erfindung darin, ein Erzeugnis, ein Verfahren und eine Verwendung dieses Erzeugnisses anzugeben, welche die verbleibende Zeitspanne zwischen dem Zeitpunkt, da der Sauerstoff den Flugzeuginsassen zugänglich gemacht wird, bis zum Zeitpunkt, da sich das Flugzeug auf einer sicheren Flughöhe entsprechend einer für Menschen überlebensfähigen Dichtehöhe befindet, soweit verlängern, dass den Piloten während eines Dekompressionseintritts mehr Zeit bleibt, Massnahmen zu ergreifen bzw. Entscheide zu fällen, zur Minimierung der Risiken und Erhöhung der allgemeinen Flugsicherheit. Zudem ist es die Aufgabe der Erfindung, ein Erzeugnis, Verfahren sowie eine Verwendung des Erzeugnisses anzugeben, damit eine Flugstrecke unter Umständen keine gegenüber dem Triebwerkausfallszenario erhöhten routentechnischen Auflagen erfüllen muss, sodass ein Flugzeug bei Dekompressionseintritt dadurch weniger schnell absinken muss, was aus routentechnischen Gründen vorteilhaft ist, da direktere Flugstrecken über erhöhtes Terrain möglich werden. Überdies ist es eine Aufgabe der Erfindung, ein Erzeugnis, ein Verfahren sowie eine Verwendung dieses Erzeugnisses anzugeben, damit im Fall eines Druckverlusts in der Kabine die Körperfunktionalität der Flugzeuginsassen mit weniger Sauerstoff als bisher gewährleistet bleibt und dadurch die mit einer reinen Sauerstoffzufuhr einhergehenden gesundheitsschädlichen Risiken eingedämmt bzw. vermieden werden. Die Erfindung soll insbesondere Menschen in einer Situation wie sie in einem Flugzeug gegeben ist, mit wenig Möglichkeit für eine körperliche Betätigung und somit erhöhter Anfälligkeit für Probleme im Fall einer Beatmung mit reinem Sauerstoff, helfen, das Auftreten der sonst drohenden Symptome wirksam zu vermeiden.

Die Aufgabe wird zum Ersten gelöst durch ein Gasgemisch zur Sicherstellung der Beatmung von Menschen in Flugzeugen im Bedarfsfall oder generell bei Hyperventilation, *das sich dadurch auszeichnet,* dass es druckabhängig je nach Dichtehöhe zusammengesetzt ist aus 7±5% CO₂ bei 15'000ft Flughöhe mit Steigerung bis zu 17±5% CO₂ bei 30'000ft Flughöhe, zur Wirkung als Bioenhancer und damit zur Verbesserung der Bioverfügbarkeit von Sauerstoff im Körper, entweder nach Zugabe zu reinem O₂ oder nach Zugabe zu einem aus einem Anteil N₂ und einem Anteil O₂ zusammengesetzten Gasgemisch für die Beatmung.

Die Aufgabe wird zum Zweiten gelöst durch ein Verfahren zum Sicherstellen der Beatmung von Menschen mit eingeschränkter Bewegungsfreiheit im Bedarfsfall oder generell bei Hyperventilation, *das sich dadurch auszeichnet,* dass ihnen Aufsetzmasken für Nase und Mund verfügbar gemacht werden, und ihnen nach dem Aufsetzen dieser Masken ein der Maske kontinuierlich zugeführtes Gasgemisch gemäss Anspruch 1 verfügbar gemacht wird.

Zum Dritten wird diese Aufgabe gelöst durch die Verwendung eines Gasgemisches nach einem der Ansprüche 1 bis 2 zur Sicherstellung der Beatmung von Menschen in Flugzeugen im Bedarfsfall, d.h. unter anderem bei Druckabfällen im Flugzeug.

Anhand der nachfolgenden Erläuterungen werden das Erzeugnis, das Verfahren sowie die Verwendung dieses Erzeugnisses offenbart und erläutert. Anhand von zahlreichen Versuchen und Messungen wurde die Effizienz des Verfahrens nachgewiesen. Dies wird nachfolgend inhaltlich diskutiert.

Es zeigt:
- Figur 1 :: Eine modifizierte Flugroute aufgrund des Hochterrains des bolivianisch-argentinischen Andengebirges;
- Figur 2 :: das zugehörige Höhenprofil der Flugroute mit erforderlichem minimalen Bodenabstand, welcher dem Höhenprofil überlagert eingezeichnet ist;
- Figur 3 :: Ein typisches Not-Abstiegsprofil eines Flugzeuges hin zu einer reduzierten Flughöhe;
- Figur 4 :: Eine Flugroute mit Fluchtwegsystem zwischen La Paz und San Salvador de Jujuy als Beispiel;
- Figur 5 :: Vorgehensweise mit Optionen A, B, C bei einem Abstieg in einem Triebwerkausfallszenario;
- Figur 6 :: Ein 737-700 CFM56-7 Notabstiegsprofil bei Druckabfall in der Kabine gemäss Boeing, für einen Standard-Abstieg in 15 Minuten bzw. einen optionalen Abstieg in 22 Minuten, im Einklang mit den ICAO-Regeln;
- Figur 7 :: Abstiegsmöglichkeiten gemäss eines 12-Minuten-Abstiegsprofils für ein Flugzeug in der Hochgebirgsgegend um La Paz;
- Figur 8 :: Abstiegsmöglichkeiten gemäss eines 22-Minuten-Abstiegsprofils für ein Flugzeug in der Hochgebirgsgegend um La Paz.

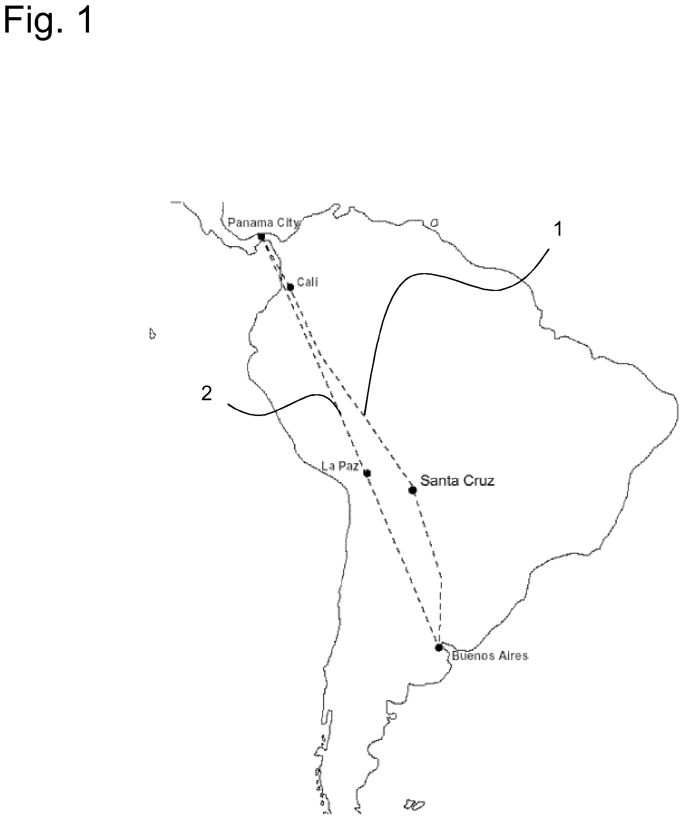

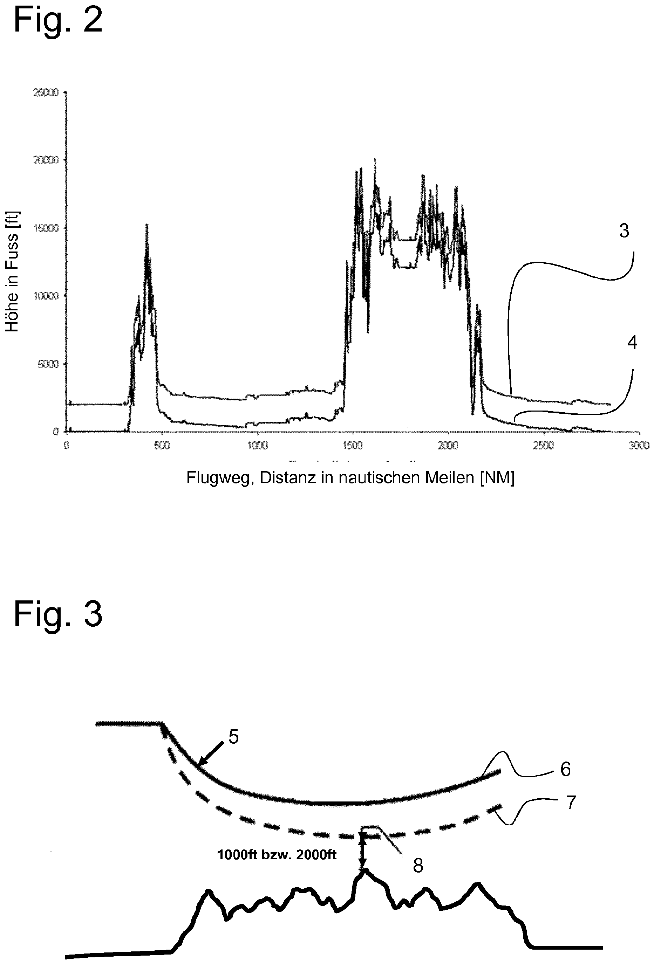

Die Figur 1 zeigt die Implikationen der Sauerstoffversorgungs-Problematik für die Routenplanung anhand einer Flugroute zwischen Panama City und Buenos Aires. Im Fall der Direktroute müsste ab etwa der Hälfte der Strecke das bolivianische und später argentinische Andengebirge überflogen werden. Dieses grossflächige, überaus hochgelegene Gebirgsterrain stellt entsprechende Anforderungen an die Abstiegsmöglichkeiten in Notfallszenarien, denn über dem weitläufigen, hohen Plateau wäre bei einem Druckabfall ein hinreichend schneller Abstieg auf eine sichere Höhe, in der die Flugpassagiere wieder eigenständig atmen können, unmöglich. Deshalb wird in der Praxis auf eine wesentlich längere Umweg-Route 1 via Santa Cruz ausgewichen, die am kritischen Andengebirge vorbei hauptsächlich über Tiefland führt. Es muss somit auf die ökonomische und effiziente Variante eines Direktflugs 2 verzichtet werden. Das bei einer direkten Route 2 zwischen Panama City und Buenos Aires zughörige Höhenprofil ist in Figur 2 dargestellt. Im Diagramm ersichtlich sind dabei zwei Kurven 3 und 4, wobei die untere 4 das geographische Höhenprofil angibt. Ihr überlagert ist der minimale erforderliche Bodenabstand 3, welcher ein Flugzeug in jedem Punkt der Flugstrecke einhalten können muss. Die jeweils vorgeschriebenen Abstiegsprofile müssen daher zwingend oberhalb der erforderlichen Abstandslinie zum Boden verlaufen.

In Figur 3 ist ein typisches Abstiegsprofil für einen notfallmässigen Abstieg aufgrund eines Triebwerkausfalls dargestellt. Ersichtlich ist sowohl die eigentliche Flugbahn 6 (*net flight path*) wie auch die idealisierte, bei der Flugroutenplanung entwickelte Flugbahn 7 (*gross flight path)*. Bei Triebwerkausfall auf einem Streckenflug muss nach einem Sinkflug 5 (*drift-down)* ein positiver Steigfluggradient erreicht werden, wenn sich das Flugzeug minimal 1'500ft über dem (Not-) Landeplatz befindet. Der positive Steiggradient ist in Figur 3 am tiefsten Punkt 8 der Flugbahn (mind. 2000ft Vertikalabstand zum überflogenen Gelände bzw. 1000ft Vertikalabstand im *level-off* Segment von niedrigeren Flugrouten) angezeigt. Ohne dass ein voll beladenes Verkehrsflugzeug die dargestellten Standards auf jedem Abschnitt seiner Flugstrecke erfüllen kann, darf es die spezifische Route nicht befliegen.

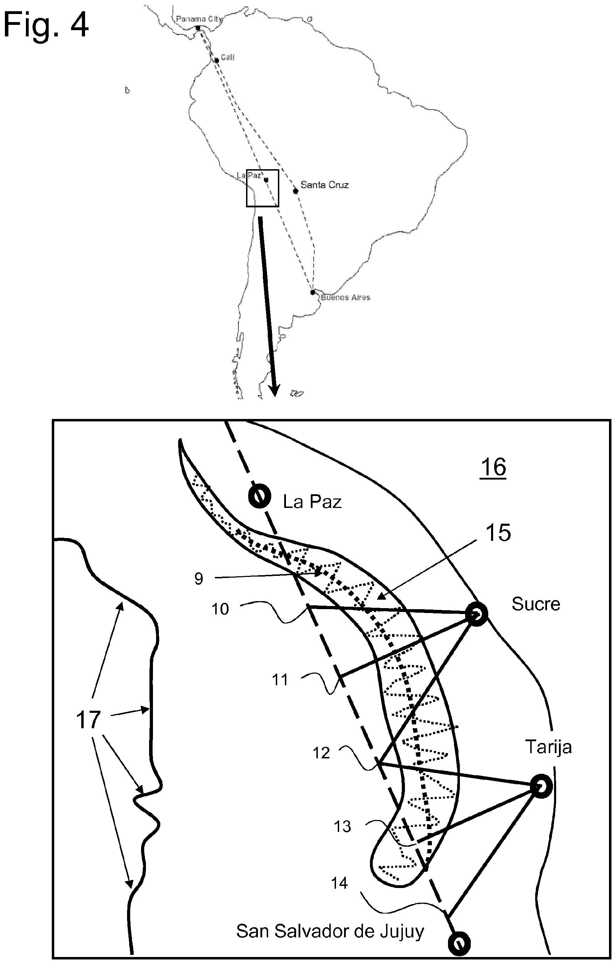

Entsprechend den Sicherheitsvorschriften wurde ein Fluchtwegsystem für alle Flugrouten über hochgelegene Territorien entwickelt. Ein solches ist am Beispiel des Andengebirges für die Flugroute zwischen La Paz und San Salvador de Jujuy in der Figur 4 eingezeichnet. Der mit punktierter Zick-Zack Linie ausgefüllte Bereich 15 kennzeichnet sehr hoch gelegenes und der Bereich 16 rechts der durchzogenen Linie sehr tief gelegenes Gelände, während die restlichen weissen Flächen Gebiete mässiger Höhe kennzeichnen. Der Gebietsstreifen 15 entlang der Flugstrecke stellt das besonders hohe Gebirgsmassiv der Bolivianisch-Argentinischen Andenkette dar. Bei der Routenplanung massgebend ist die eingangs erwähnte begrenzte Menge an Sauerstoff in der Kabine, um Crew und Passagiere damit zu versorgen. Eine Route, die längs dieses Gebirgsmassivs und daher ständig über dasselbe führen würde, wäre deshalb nicht zulässig. Dies ist in der Figur 4 durch die punktierte, starke Linie 9 angezeigt. Stattdessen kann zum Beispiel die strichlinierte, direkte Route von La Paz nach San Salvador de Jujuy beflogen werden. Lokale bzw. flugabhängige Faktoren wie Wind, Temperatur, Lokaldruck, Gewicht etc. können ausserdem diese Route in der Praxis etwas abweichen lassen und sind daher spezifisch auszumachen. Es sei an dieser Stelle erwähnt, dass Flugzeuge, die eine so spezifische Route wie diese strichlinierte eingezeichnete Gerade befliegen, mit entsprechenden Hochkapazitäts-Sauerstoff-Notfall-Systemen ausgerüstet sind, wie im nachfolgenden Abschnitt erläutert. Auf der Direktroute zwischen La Paz und San Salvador de Jujuy erkennbar sind zwei Kehrpunkte 11 und 13 angegeben, welche auf kürzestem Weg zu den Flughäfen von Sucre bzw. Tarija führen. Die möglichen Fluchtwege sind hier einmal rechtwinklig zur Direktroute (kürzeste Fluchtwege) und einmal als gleiche Seiten eines gleichschenkligen Dreiecks (längste Fluchtwege) eingezeichnet. Dies dient der Orientierung, um die Punkte für den schnellstmöglichen Abstieg (*ideal turn points*) zu identifizieren. Exakt zwischen diesen sogenannten *ideal turn points* liegen die jeweils kritischen Punkte 10, 12 und 14, welche sowohl die eine wie die andere Landemöglichkeit in Frage kommen lassen. Welche Route bei einer Notfall-Landung letztlich gewählt wird, liegt im Ermessen des Flugkapitäns.

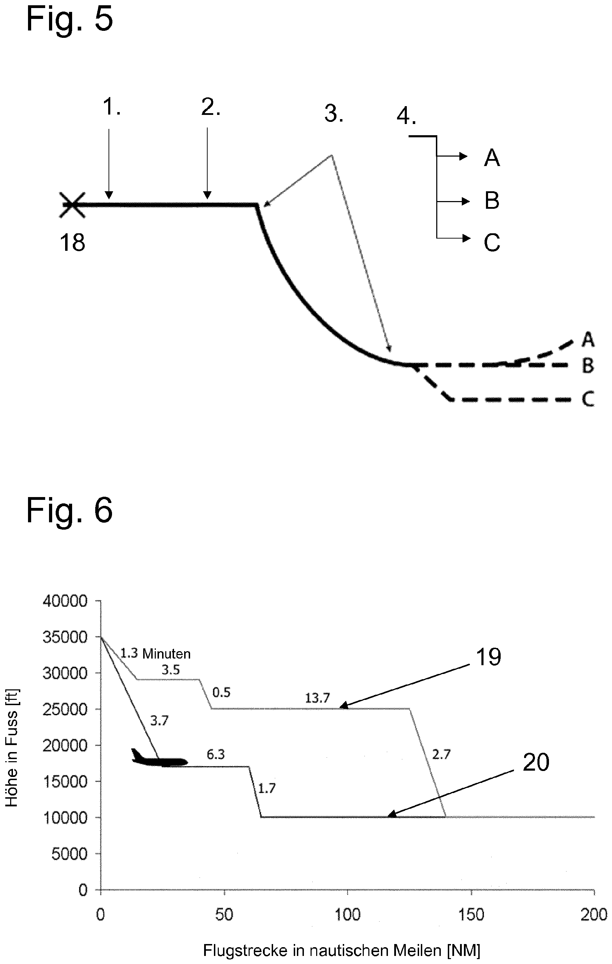

Einige Passagierflugzeuge und Business Jets besitzen Hochkapazitäts-Sauerstoff-Notfall-Systeme, auch bekannt als *burning systems* (aufgrund der Wärmeerzeugung infolge der chemischen Reaktion, mittels welcher Sauerstoff an Bord erzeugbar ist). Entsprechend sind einige wenige Linienflugzeuge, die weite Strecken über Hochgebirge zurücklegen, mit solchen Hochkapazitäts-Sauerstoffgeräten ausgerüstet. Allerdings ergeben die damit verbundenen Sauerstofftanks und nötigen Installationen zusätzliches Gewicht, was auf Kosten der Flugleistung geht - erst recht bei einem Triebwerkausfall, wenn der Schub nur noch von einem Teil der Triebwerke stammt. Die in einem solchen Szenario sich effektiv ergebende *OEI* (*one-engine inoperable*) *service ceiling* hängt von einer Reihe von Faktoren ab, unter anderem von der Anzahl verbleibender funktionstüchtiger Triebwerke und insbesondere auch vom Gewicht des Flugzeugs. Bei einem Notsinkflug hat die Besatzung das Vorgehen diesen Umständen genauestens anzupassen. In Figur 5 werden die möglichen Optionen A, B und C angegeben. In jedem Fall wird als 1. Massnahme unverzüglich nach Aussetzen eines Triebwerks beim Punkt 18 der maximale, kontinuierlich haltbare Schub *(maximum continuous thrust)* eingestellt, als 2. Massnahme wird verlangsamt und als 3. Massnahme wird mit einer definierten drift-down-Geschwindigkeit der Notsinkflug eingeleitet. Je nach Situation entscheidet man sich als 4. Massnahme eine von drei Optionen A, B oder C durchzuführen. Nach Option A wird nach dem *drift-down* die Fluggeschwindigkeit beibehalten und mit anhaltendem Treibstoffverbrauch einen kontinuierlich höheren Fluglevel eingenommen. Nach Option B wird die Flughöhe während der verbleibenden Flugzeit beibehalten und allmählich auf Reisegeschwindigkeit bei Triebwerkausfall *(engine-out long -range cruise speed)* beschleunigt, oder aber nach Option C wird abgesunken und sofort auf die Reisegeschwindigkeit bei Triebwerkausfall *(engine-out long-range cruise speed)* beschleunigt. Kann aufgrund des hochgelegenen Geländes die erforderliche Höhe nach einem *drift-down,* also mindestens die Höhe von C, auf dem weiteren Flugweg nicht eingenommen werden, muss das Ladegewicht des Flugzeuges reduziert werden, etwa durch partielle Entleerung oder *burn off* der Treibstofftanks, wodurch eine höhere Flughöhe einnehmbar ist (Option A). Dies jedoch setzt voraus, dass ein Notlandeplatz in absehbarer Entfernung vorhanden ist. Das Gewicht eines Flugzeugs wirkt sich im Triebwerkausfall immer negativ aus. Zwischen der Lösung von Beatmungsproblemen bei Druckabfall und den erforderlichen Abstiegsszenarien bei Triebwerkausfall besteht ein Zielkonflikt. Ist aufgrund einer bestimmten Flugstrecke die Sauerstoffversorgung der Flugzeuginsassen vorrangig, wird der Gewichtsnachteil durch eine leistungsfähigere Sauerstoffeinrichtung in Kauf genommen. Die im Druckabfallszenario dadurch zusätzlich gewonnene Zeit pro Höhenmeter erlaubt grössere Distanzen für Fluchtrouten, wodurch mehr Fluchtwege erschlossen werden können. Ist der Gewichtsnachteil dagegen nicht mehr verkraftbar, kann die Strecke nicht beflogen werden, wie das für ausgedehnte Strecken über Hochgebirge in aller Regel der Fall ist.

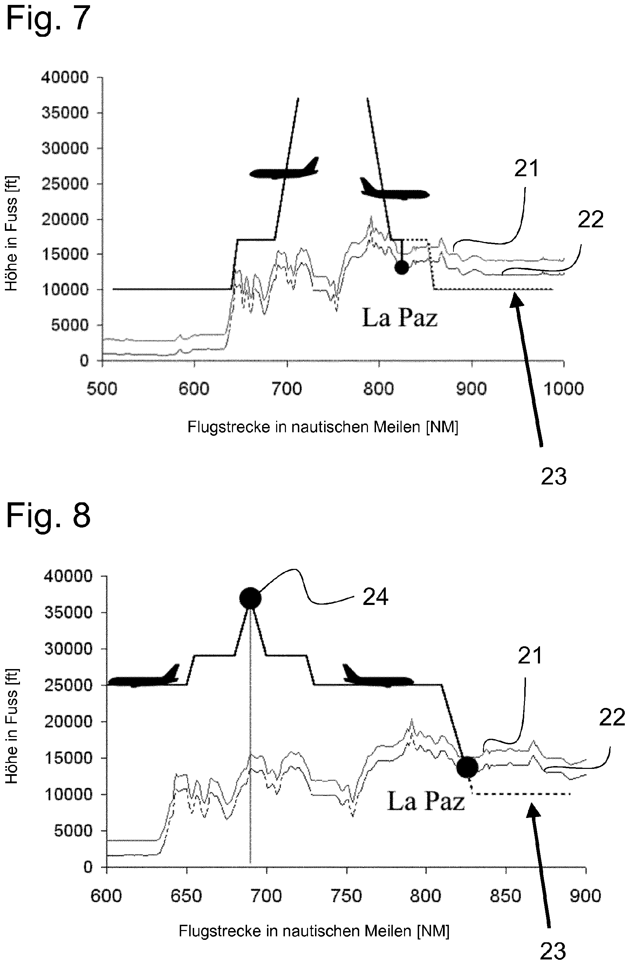

Bei einem Dekompressionsvorfall in der Flugzeugkabine gelten die spezifischen Vorschriften der ICAO. Ausgehend davon leiten sich die spezifischen Sinkflugprofile der verschiedenen Flugzeughersteller bzw. Fluggesellschaften ab. Grundsätzlich schreibt die ICAO je zwei verschiedene Sinkflugprofile vor, ein Standard-Abstiegsprofil sowie ein optionales Abstiegsprofil für Ausnahmestrecken. Solche zwei Profile sind in Figur 6 mit Boeing-spezifischen Werten gezeigt, nämlich ein 12-Minuten Standard-Profil 20 sowie ein optionales 22-Minuten Profil 19. Die Grössenordnung dieser Werte ist für alle Flugzeughersteller bzw. Fluggesellschaften dieselbe. Nach einem Dekompressionsvorfall muss ein Flugzeug des Herstellers Boeing (The Boeing Company) entsprechend seiner Zulassung in 12, respektive in 22 Minuten auf 14'000ft abgesunken sein. Dabei sind die in den jeweiligen Profilen 19, 20 angegebenen Zwischenhöhen und Zwischenzeiten zu berücksichtigen. Eine Flugstrecke muss so gewählt werden, dass diese Höhen und Zeiten jederzeit eingehalten werden können. Beim Überfliegen ausgedehnter Gebirgszüge müssen dabei Umwege in Kauf genommen werden, um bei einem Dekompressionsvorfall jederzeit das vorgeschriebene Sinkflugprofil anwenden und damit rasch genug sinken zu können. Direktere Flugrouten über ausgedehnte Gebirgszüge können heute fast ausschliesslich von Frachtflugzeugen beflogen werden, weil diese mehr Notsauerstoff für die Besatzung mitführen als dies bei Linienflugzeugen möglich ist. Der geplante Flugweg wird, wie vorgängig beschrieben, ebenfalls durch einen potentiellen Triebwerkausfall bestimmt, wobei das Abstiegsprofil 7 in Figur 3 massgeblich ist. Moderne Linienflugzeuge sind nach einem Triebwerkausfall zwar in der Lage, deutlich höhere Flughöhen zu fliegen, als durch die Profile bei Dekompressionsvorfällen vorgegeben wird. Folglich sind die Beschränkungen bei der Wahl der Flugroute in erster Linie durch einen potentiellen Kabinendruckverlust mit den Notsinkflugprofilen 19, 20 entsprechend der Figur 6 gegeben. Grundsätzlich kommen alle bei Druckverlust möglichen Fluchtwege auch bei einem Triebwerkausfall in Frage, umgekehrt jedoch muss eine im Triebwerkausfall infrage kommende Fluchtroute den zeitlichen Rahmenbedingungen des Notsauerstoffvorrats an Bord genügen. Dadurch wird die Anzahl potentieller Fluchtwege erheblich reduziert. Deshalb sind hochgelegene Gebiete wie die zentralasiatische Gebirgslandschaft oder die Anden nur beschränkt für den Flugverkehr mit Passagiermaschinen freigegeben. Dabei zeigt Figur 7, wie ein Flugzeug mit einer Zulassung entsprechend seiner Sauerstoffversorgung für einen Sinkflug innert 12 Minuten in einem Notfallszenario über den Bolivianischen Anden absteigen kann. Das in seinem Fall vorgeschriebene 12-Minuten Profil verunmöglicht eine Landung des Flugzeugs in La Paz aufgrund der erforderlichen Zwischenhöhen und Zwischenzeiten dieses Profils. Letzteres wird durch die gestrichelte Linie 23 für das östlich der Stadt La Paz verlaufende Sinkprofil verdeutlicht, wobei diese Linie 23 unterhalb des minimal erforderlichen Bodenabstands 21 und sogar unterhalb des geografischen Höhenprofils 22 verläuft. Möglich ist ein Abstieg daher nur in westlicher Richtung, sofern das Flugzeug eine Position wie auf der entsprechenden Sinkfluglinie angegeben oder aber weiter westlich davon voraussetzt. Aus diesem Grund ist es Flugzeugen mit einer Zulassung für einen Notfallabstieg in 12 Minuten nicht möglich, eine Route über La Paz zu fliegen, wie durch das westlich positionierte Flugzeug in der Figur 7 angezeigt. Hingegen wird ein Flugzeug, welches diese Region überfliegen können muss, mit einem Hochkapazitäts-Sauerstoffversorgungssystem ausgerüstet sein, damit es bei Druckabfall gemäss seiner Zulassung in 22 Minuten absteigen kann. Auch in einem solchen Fall müssen die Zwischenhöhen und Zwischenzeiten eingehalten werden. Ein kritischer Punkt 24, bei dem diese Anforderungen bei einer Notlandung in La Paz nicht mehr erfüllt werden können, ist in der Figur 8 eingezeichnet. Befindet sich ein Flugzeug mit Zulassung für ein 22-Minuten Profil auf der Höhe oder gar unterhalb des kritischen Punkts 24, kann es nur einen Fluchtweg westwärts einschlagen, da im andern Fall das Gebirgsmassiv östlich von La Paz im Wege steht bzw. das Sinkprofil 23 unterhalb des minimal erforderlichen Bodenabstands 21 und sogar unterhalb des geografischen Höhenprofils 22 verläuft. Zusammenfassend kann gesagt werden, dass sich durch die Druckabfallproblematik viele Einschränkungen im Flugverkehr ergeben.

Zwar geht man im herkömmlichen Druckabfallszenario von vollkommen funktionstauglichen Triebwerken aus, wodurch im Prinzip höhere Geschwindigkeiten möglich sind als bei einem Triebwerkausfall. Man würde folglich erwarten, dass Hindernisse in Form von hochgelegenem Terrain innerhalb des vorgeschriebenen Zeitintervalls eher überflogen werden könnten, was im Ergebnis weniger Einschränkungen bei der Routenplanung bedeutet. In der Realität jedoch muss im Fall eines Druckverlusts zunächst von einem strukturellen Ausfall ausgegangen werden, wodurch die Fluggeschwindigkeit sofort angepasst, also verringert wird. Daher kann ausgedehntes Hochterrain nicht uneingeschränkt unter gleichzeitiger Einhaltung der oben erwähnten Abstiegsprofile beflogen werden.

Bei gesunden Menschen kann eine Sauerstoffuntersättigung des Gewebes vor allem auf eine O₂-arme Umgebung zurückgeführt werden. Das wohl grösste Risiko eines akuten Sauerstoffmangels für einen durchschnittlichen, gesunden Menschen besteht im Fall eines Druckabfalls in einem Flugzeug. Fällt im Flugzeug der Kabinendruck auf grossen Flughöhen unerwartet ab, kommt es aufgrund des geringen Partialdrucks zu einer Sauerstoffunterversorgung des Gewebes (Hypoxie). Eine Hypoxie kann zu schweren Organschädigungen mit unter Umständen tödlichem Ausgang führen. Eine tückische Gefahr der Hypoxie besteht darin, dass sie durch die Betroffenen selbst nicht immer bzw. zu spät erkannt wird und der Betroffene in seiner Handlungsfähigkeit eingeschränkt ist, ehe er Gegenmassnahmen treffen kann. Als Symptome einer Hypoxie gelten falsche Selbsteinschätzung, Euphorie, Müdigkeit, Orientierungsverlust bis hin zu Bewusstlosigkeit. In der Luftfahrt ist Hypoxie ein äusserst ernst zu nehmender körperlicher Zustand, welcher schwerwiegende Folgen haben kann, speziell für die Besatzung eines Flugzeuges.

Bei der Versorgung eines Menschen mit 100%igem Sauerstoff wird der O₂-Partialdruck auf den fünffachen Wert erhöht. Nach dem Gasgesetz von Henry ist der Partialdruck eines Gases über einer Flüssigkeit proportional zur Konzentration des (physikalisch) gelösten Gases in dieser Flüssigkeit. Bei einer Versorgung mit reinem Sauerstoff erhöht sich daher der Anteil des gelösten Sauerstoffs im Blut um das Fünffache. Für den chemisch an das Hämoglobin der roten Blutkörperchen gebundenen Sauerstoff findet das Gasgesetz hingegen keine Anwendung. Bei normaler Atemluft beträgt die Sauerstoffsättigung des Blutes zwischen 95-100%. Somit wird bei der Beatmung vorwiegend der Anteil des in physikalischer Form gelösten Sauerstoffs angereichert, der entsprechend druckabhängig ist. Atmet der Mensch reinen Sauerstoff in einem Umgebungsdruck von 2.5 bar ein, löst sich - verglichen mit Normalbedingungen - die 20fache Menge an Sauerstoff im Blut. Die sog. systematische hyperbare Oxygenierungstherapie (Sauerstoffüberdrucktherapie) wird angewendet, wenn Sauerstoffmangel im Gewebe des Patienten den Heilungsvorgang behindert oder aber wenn Sauerstoff bei Notfallsituationen als lebensrettende Massnahme zugeführt werden muss. Bisher hat die hyperbare Oxygenierung aber keine breite klinische Anwendung gefunden, was vor allem auf die Nebenwirkungen infolge des hohen Sauerstoffgehalts sowie des Überdrucks zurückzuführen ist. Die intensivmedizinische Überdruckbeatmung zählt zu den Hauptursachen für sauerstofftoxische Schäden.

Der Druck der Atemluft ist ein stark regulierender Parameter. Er kann bei der Sauerstoffanreicherung im Blut auch mässigenden Einfluss ausüben. Wenn im Fall einer anhaltenden, kontrollierten Beatmung reiner Sauerstoff verwendet wird, wie z.B. in der Raumfahrt, drosselt man den Umgebungsdruck erheblich, auf etwa 0.3 bar. Somit lässt sich der Luftdruck und damit der Sauerstoff-Partialdruck der zugeführten Atemluft reduzieren (vgl. mit dem Sauerstoff-Partialdruck von 0.21 bar bei Normaldruck). Bei einer anhaltenden Beatmung mit reinem Sauerstoff beginnt bereits über besagtem Wert von 0.3 bar das Risiko für eine Sauerstofftoxikose.

Zwar kann die Beatmung mit reinem Sauerstoff durch Variation des Umgebungsdruckes für verschiedene Szenarien ermöglicht werden, aber aufgrund der sich konkurrenzierenden Eigenschaften kann allein der Sauerstoff nicht nebenwirkungsfrei in der Dosis zugeführt werden, derer es aufgrund des Sauerstoffmangels bedarf. In Versuchen und Testreihen wurde nun überraschenderweise festgestellt, dass durch das Einatmen eines Gasgemisches aus 7±5% CO₂ bei 15'000ft Flughöhe mit Steigerung bis zu 17±5% CO₂ bei 30'000ft Flughöhe beispiellose Verbesserungen der körperlichen und geistigen Funktionsfähigkeit bei akuter Sauerstoffuntersättigung des Körpers erzielt werden, verglichen mit der reinen Sauerstoffzufuhr.

Kommt es zu einer Sauerstoffunterversorgung des Körpers, reagiert dieser mit einer Beschleunigung der Atemfrequenz. Durch die Erhöhung der Atemfrequenz wird nun mehr Sauerstoff pro Zeiteinheit eingeatmet, es wird jedoch gleichzeitig auch mehr Kohlenstoffdioxid ausgeatmet. Kohlendioxid ist im Körper als Kohlensäure (H₂CO₃) gebunden. Aus der chemischen Gleichgewichtsformel 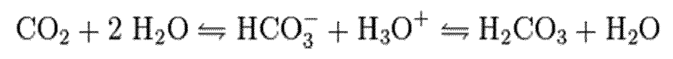 wird ersichtlich, dass bei einer Abnahme von CO₂ im Körper sich entsprechend die Anzahl H₃O⁺ - Ionen im Blut reduziert. Dadurch kommt es zu einer Verschiebung des Säure-Basen-Gleichgewichts, denn das Blut wird zunehmend basischer. Im Extremfall führt dies zu einer respiratorischen Alkalose mit Symptomen wie Muskelkrämpfen, Bewusstseinseinschränkungen bis hin zur Bewusstlosigkeit. Auch wird durch den Anstieg des pH-Wertes im Blut die Konzentration des frei gelösten ionisierten Calciums verringert (Hypokalzämie), was zu Übererregbarkeit von Muskulatur und Nervensystem mit spasmischen Symptomen führt. Eine erhöhte CO₂-Konzentration im Blut führt umgekehrt dazu, dass sich der pH-Wert des Blutes in den sauren Bereich verschiebt. An den Gefässen vieler Organe befinden sich CO₂-Rezeptoren. Je nach Organ kontrahieren oder erweitern sich die Blutgefässe unter dem Einfluss von CO₂. Im Gehirn weiten sich die Gefässe bei einer erhöhten CO₂-Konzentration. Dadurch wird die Blut-Durchflussmenge gesteigert und mit ihr auch die Sauerstoffmenge, welche pro Zeiteinheit die Zellen erreicht. Der Körper versucht auf diese Weise die Sauerstoffunterversorgung zu kompensieren und das Gehirn solange wie möglich mit genügend Sauerstoff zu versorgen. Der gegenteilige Effekt wird beobachtet, wenn der Körper mit hochdosiertem O₂ versorgt wird, unter gleichzeitiger Abnahme des CO₂. Eine Hypokapnie, also ein erniedrigter CO₂-Partialdruck im arteriellen Blut, führt im Gehirn zu einer Kontraktion der Blutgefässe und somit zu einer Verringerung der Blut- und Sauerstoffzufuhr. In der Situation einer Dekompression im Flugzeug kommt es zu einer Unterversorgung des Körpers mit Sauerstoff. Der Körper beginnt zu hyperventilieren. Selbst wenn der Flugzeuginsasse relativ schnell zu den Notsauerstoffmasken greift, wird durch die stressverursachenden Umstände die Tendenz zur Hyperventilation noch weiter gefördert. Durch die Hyperventilation wird die Ausatmung von Kohlendioxid beschleunigt. Dadurch sinkt der Kohlendioxid-Anteil im Körper. Da sich in der beschriebenen Situation der beatmete Mensch körperlich wenig bewegt, wird in den Muskelzellen weniger Kohlendioxid gebildet und der Effekt des Kohlendioxid-Mangels noch entsprechend beschleunigt. Bei Flugzeuginsassen, die zumeist und gerade in Notsituationen an ihre Sitze gebunden sind, kann diese Einschränkung der Bewegung schwere Folgen haben, weil der Körper dann weniger Kohlendioxid produziert. Der speditive Abstieg auf eine sichere Flughöhe ist nicht zuletzt deshalb lebenswichtig.

Wird dem Atemgas wie eingangs erwähnt eine dosierte, druckabhängige Menge CO₂ zugesetzt, so kann den Auswirkungen, wie im vorangehenden Abschnitt beschrieben, Einhalt geboten werden. Weil dem Körper aktiv Kohlendioxid zugeführt wird, um die Blutgefässe im Gehirn zu entspannen, geht die Sauerstoffversorgung des Körpergewebes bei gleichzeitig reduzierter Sauerstoffmenge effizienter vonstatten. Damit wird der Sauerstoff schneller und in einem grösseren Umfang resorbiert und dem Gewebe bzw. den Zellen zur Verfügung gestellt. Das erfindungsgemässe Gasgemisch zur Sicherstellung der Beatmung im Notfall erhöht die Bioverfügbarkeit von Sauerstoff, namentlich die orale Bioverfügbarkeit, weil Kohlendioxid aufgrund seiner Wirkungsweise in abgestimmten Dosen als Bioenhancer wirkt. Letztlich wird der Körper dank des erfindungsgemässen Gasgemisches mit einer Teilmenge herkömmlicher Sauerstoffdosen und über wesentlich längere Zeiträume auf einem physiologischen Kohlendioxidgehalt gehalten, was insbesondere im Fall eines Druckabfalls in der Flugzeugkabine grosse Vorteile schafft.

Flugmedizinische Versuche haben überraschend aufgezeigt, dass es möglich ist, durch das Atmen einer mit CO₂ angereicherten Luft die nach den Luftfahrtnormen vorgegebenen Werte zu erreichen: 84% Sauerstoffsättigung im Blut sind für eine kurze Überbrückungszeit von maximal einer Minute vorgeschrieben, und 90% für eine längere Überbrückungszeit von mehr als einer Minute. Probanden wurde auf verschiedenen Dichtehöhen jeweils so viel CO₂ verabreicht, dass sich in ihrem Blut ein physiologischer CO₂-Partialdruck von 40mmHg einstellte. Dabei wurde die Atemluft auf der Dichtehöhe 15'000ft mit 8% CO₂, auf 20'000ft mit 11% CO₂ und auf 30'000ft mit 16.5% CO₂ angereichert. Diese Anreicherung geschah auf Kosten des Stickstoffes. Folglich setzten sich die Gasgemische für die künstliche Atemluft insgesamt wie folgt zusammen:
Auf 15'000ft Dichtehöhe: 21% O₂, 8% CO₂, 71% N₂
Auf 30'000ft Dichtehöhe: 21% O₂, 16.5% CO₂, 62.5% N₂

Jeder Proband erlebte zweimal einen simulierten Notabstieg aus 37'000ft Höhe auf 10'000ft hinunter mit den von der ICAO vorgegebenen Profilen. Einmal atmete er die bisher üblichen 100% O₂, das zweite Mal das oben angegebene Gasgemisch mit CO₂-Anreicherung. Das Experiment erfolgte mit einem randomisierten, doppelblinden Protokoll. Weder der Untersucher noch der Proband wussten, welches Gas die Probanden in welchem Abstieg atmeten. Die Befunde lassen erkennen, dass folgende entscheidende Vorteile realisierbar sind:
1. Die mitzuführende Menge an Kabinensauerstoff kann reduziert werden.
2. Es können aufgrund angepasster *drift-down procedures* direktere Flugrouten geflogen werden und damit ganz wesentlich Kosten und Zeit eingespart werden.
3. Weil nunmehr eine geringere Treibstoffmenge im Flugzeug mitgeführt werden kann, vergrössert sich die Ladekapazität des Flugzeugs.
4. Durch den niedereren Treibstoffverbrauch wird die Umwelt geschont.

Eine besonders elegante Komponente am Gesamtkonzept des erfindungsgemässen Verfahrens der CO₂-Zudosierung liegt darin, dass ein Passagier sein benötigtes CO₂ bzw. O₂ zum Teil selbst produziert. Beim normalen Einatmen unter Normaldruck ist die Atemluft zusammengesetzt aus ca. 78% Stickstoff (N₂), ca. 21% Sauerstoff (O₂) und ca. 1% Restgase. Beim Ausatmen misst man ca. 78% Stickstoff (N₂), 16% Sauerstoff (O₂), 4% Kohlendioxid (CO₂) und ca. 2% Restgase. Dieses Kohlendioxid wie auch der Sauerstoff können rekuperiert werden. Somit kann bei der Zudosierung des erfindungsgemässen Gasgemisches ein Anteil des Kohlendioxids und des Sauerstoffs direkt von der zu beatmenden Person geliefert werden und wird dann von ihr wieder eingeatmet, und der Rest wird künstlich zudosiert, und zwar je höher die Dichtehöhe ist, umso mehr CO₂ muss auf Kosten des Stickstoffes zudosiert werden.

### Ziffernverzeichnis

- 1: Effektive Flugroute
- 2: Nicht befliegbare Direktroute
- 3: Minimaler erfoderlicher Bodenabstand
- 4: Geografisches Höhenprofil
- 5: Sinkflug *(drift-down)*
- 6: Brutto Flugweg *(gross flight path)*
- 7: Netto Flugweg *(net flight path)*
- 8: Erreichen des positiven Steigfluggradienten *(positive climb gradient begins)*
- 9: Nicht befliegbare Strecke
- 10: Kritischer Punkt 1 *(critical point 1)*
- 11: Idealer Wendepunkt 1 *(ideal turn point 1)*
- 12: Kritischer Punkt 2 *(critical point 2)*
- 13: Idealer Wendepunkt 2 *(ideal turn point 2)*
- 14: Kritischer Punkt 3 *(critical point 3)*
- 15: Hochland
- 16: Tiefland
- 17: Südamerikanische Westküste
- 18: Triebwerkausfall *(engine failure)*
- 19: Optionales 22-Minuten System *(Optional 22 Minute System)*
- 20: Standard 12-Minuten System *(Standard 12 Minute System)*
- 21: Minimaler erforderlicher Bodenabstand
- 22: Geografisches Höhenprofil
- 23: Verlauf des Abstiegsprofils unterhalb des geographischen Höhenprofils 22
- 24: Kritischer Punkt

### Checklistenpunkte (Figur 5)

1. Einstellen des maximalen dauerhaften Schubs *(MCT* = *maximum continuous thrust)*
2. Höhe halten, verlangsamen bis Sinkfluggeschwindigkeit erreicht ist
3. Halten der Sinkfluggeschwindigkeit
4. Auswahl unter drei Absink-Optionen *(drift down options):*
   A: Fluggeschwindigkeit halten und steigen bis Treibstoff verbraucht ist
   B: Höhe halten und beschleunigen zur Reisegeschwindigkeit bei Triebwerkausfall *(EOLRC* = *engine-out long-range cruise speed)*
   C: Sinken und sofort beschleunigen zur Reisegeschwindigkeit bei Triebwerkausfall *(EOLRC* = *engine-out long-range cruise speed)*

## Patentansprüche

1. Gasgemisch zur Sicherstellung der Beatmung von Menschen in Flugzeugen im Bedarfsfall oder generell bei Hyperventilation, ***dadurch gekennzeichnet,* dass** es druckabhängig je nach Dichtehöhe zusammengesetzt ist aus 7±5% CO₂ bei 15'000ft Flughöhe mit Steigerung bis zu 17±5% CO₂ bei 30'000ft Flughöhe, zur Wirkung als Bioenhancer und damit zur Verbesserung der Bioverfügbarkeit von Sauerstoff im Körper, entweder nach Zugabe zu reinem O₂ oder nach Zugabe zu einem aus einem Anteil N₂ und einem Anteil O₂ zusammengesetzten Gasgemisch für die Beatmung.

2. Gasgemisch nach Anspruch 1, ***dadurch gekennzeichnet,* dass** es druckabhängig je nach Dichtehöhe zusammengesetzt ist aus 7±5% CO₂ bei 15'000ft Flughöhe mit linearer Steigerung bis zu 17±5% CO₂ bei 30'000ft Flughöhe.

3. Verfahren zum Sicherstellen der Beatmung von Menschen mit eingeschränkter Bewegungsfreiheit im Bedarfsfall oder generell bei Hyperventilation, ***dadurch gekennzeichnet,* dass** ihnen Aufsetzmasken für Nase und Mund verfügbar gemacht werden, und ihnen nach dem Aufsetzen dieser Masken ein der Maske kontinuierlich zugeführtes Gasgemisch gemäss einem der Ansprüche 1 bis 2 verfügbar gemacht wird.

4. Verfahren nach Anspruch 3, ***dadurch gekennzeichnet,* dass** im Fall eines Druckabfalls in einem Flugzeug Aufsetzmasken für Nase und Mund verfügbar gemacht werden, und die Flugzeuginsassen nach Aufsetzen dieser Masken mit dem Gasgemisch gemäss einem der Ansprüche 1 bis 2 beatmet werden.

5. Verfahren nach Anspruch 3, ***dadurch gekennzeichnet,* dass** im Fall eines Druckabfalls in einem Flugzeug Aufsetzmasken für Nase und Mund verfügbar gemacht werden, und die Flugzeuginsassen nach Aufsetzen dieser Masken mit dem Gasgemisch gemäss einem der Ansprüche 1 bis 2 beatmet werden und gleichzeitig aus der Ausatemluft ein CO₂-Anteil und ein O₂-Anteil extrahiert wird und mit Beimischung externer Gase zu einem Gasgemisch gemäss einem der Ansprüche 1 bis 2 aufbereitet und den Flugzeuginsassen verabreicht wird.

6. Verwendung eines Gasgemisches gemäss einem der Ansprüche 1 bis 2 zur Sicherstellung der Beatmung von Menschen in Flugzeugen im Bedarfsfall, das heisst unter anderem bei Druckabfällen in einer Flugzeug-Druckkabine.

## Claims

1. Gas mixture for ensuring breathing of persons in aircrafts in case of need, or generally in case of hyperventilation, ***characterized in that*** it comprises dependent on pressure and density altitude 7±5% CO₂ at 15,000ft flight altitude, and increasing with altitude, up to 17±5% CO₂ at 30,000ft flight altitude, to act as bioenhancer and thus, to improve the bioavailability of oxygen in the body, by addition to either pure O₂ or to a gas mixture comprising a fraction of N₂ and a fraction of O₂ for ventilation.

2. Gas mixture according to claim 1, ***characterized in that*** it comprises dependent on pressure and density altitude 7±5% CO₂ at 15,000ft flight altitude, and increasing linearly up to 17±5% CO₂ at 30,000ft flight altitude.

3. Method for ensuring breathing of people with limited mobility in case of need, or generally in case of hyperventilation, ***characterized in that*** artificial respiration masks for nose and mouth are made available to them, through which the gas mixture according to one of the claims 1 to 2 is continuously supplied to them upon fitting the mask.

4. Method according to claim 3, ***characterized in that*** artificial respiration masks for nose and mouth are made available in case of loss of cabin pressure in an aircraft, and upon fitting these masks, the occupants of the aircraft are ventilated with the gas mixture according to one of the claims 1 to 2.

5. Method according to claim 3, ***characterized in that*** artificial respiration masks for nose and mouth are made available in case of loss of cabin pressure in an aircraft, and upon fitting these masks, the occupants of the aircraft are ventilated with the gas mixture according to one of the claims 1 to 2, while a fraction of CO₂ and a fraction of O₂ is extracted from the air exhaled by them, respectively, and admixtured by means of additition to external gases to yield a gas mixture according to one of the claims 1 to 2, which is administered to the occupants of the aircraft.

6. Use of a gas mixture according to one of the claims 1 to 2 for ensuring good ventilation of persons in aircrafts in case of need, that is, inter alia, in case of depressurization in a pressurised aircraft cabin.

## Revendications

1. Mélange gazeux, destiné à assurer la ventilation d'individus en cas de besoin dans des avions ou de manière générale, en cas d'hyperventilation, **caractérisé en ce qu'**il est constitué en fonction de la pression, selon la densité de 7 ± 5 % de CO₂ à une altitude de vol de 15 000 ft avec une augmentation jusqu'à 17 ± 5 % de CO₂ à une altitude de vol de 30 000 ft, pour faire effet de bioactivateur et pour améliorer ainsi la biodisponibilité d'oxygène dans le corps, soit par ajout à de l'O₂ ou après l'ajout à un mélange gazeux composé d'une part de N₂ et d'une part de O₂ pour la ventilation.

2. Mélange gazeux selon la revendication 1, **caractérisé en ce qu'**en fonction de la pression, selon la densité, il est composé de 7 ± 5% de CO₂ à une altitude de vol de 15 000 ft, avec une augmentation linéaire de jusqu'à 17 ± 5% de CO₂ à une altitude de vol de 30 000 ft.

3. Procédé, destiné à assurer la ventilation d'individus à mobilité réduite en cas de besoin, ou de manière générale, en cas hyperventilation, **caractérisé en ce qu'**on met à leur disposition des masques à poser sur le nez et sur la bouche et après la pose desdits masques, on met à leur disposition un mélange gazeux selon l'une quelconque des revendications 1 et 2, alimenté en continu dans le masque.

4. Procédé selon la revendication 3, **caractérisé en ce que** dans le cas d'une chute de pression dans un avion, on met à disposition des masques à poser sur le nez et sur la bouche, et après la pose desdits masques, on ventile les passagers de l'avion avec le mélange gazeux selon l'une quelconque des revendications 1 et 2.

5. Procédé selon la revendication 3, **caractérisé en ce que** dans le cas d'une chute de pression dans un avion, on met à disposition des masques à poser sur le nez et sur la bouche et après la pose desdits masques, on ventile les passagers de l'avion avec le mélange gazeux selon l'une quelconque des revendications 1 et 2 et on extrait simultanément à partir de l'air expiré une part de CO₂ et une part d'O₂ et en y ajoutant des gaz externes, on prépare un mélange gazeux selon l'une quelconque des revendications 1 et 2 et on l'administre aux passagers de l'avion.

6. Utilisation d'un mélange gazeux selon l'une quelconque des revendications 1 et 2 pour assurer la ventilation d'individus dans un avion en cas de besoin, c'est-à-dire en cas d'une chute de pression dans une cabine d'avion pressurisée.
